# EUROPEAN PATENT APPLICATION

(11) **EP 4 837 916 A1**
(43) Date of publication of application: **11.11.2026**
(21) Application number: 24915698.5
(22) Date of filing: 18.12.2024
(51) Int. Cl.: C07H 3/02, B01J 21/08, C07B 61/00

(54) **METHOD FOR PRODUCING GLUCOSE AND CATALYST FOR SAME**

(30) Priority: 04.01.2024 JP 2024000266
(71) Applicant: SUMITOMO CHEMICAL COMPANY, LIMITED, Tokyo 103-6020 (JP); National Institute of Technology, Hachioji-shi, Tokyo 1930834 (JP)
(72) Inventor: INOMATA, Katsuya, Tokyo 103-6020 (JP); YONEMOTO,Tetsuro, Ichihara-shi, Chiba 299-0195 (JP); GABE, Atsushi, Kurume-shi, Fukuoka 830-8555 (JP)
(74) Representative: Vossius & Partner Patentanwälte Rechtsanwälte mbB
(86) International application number: PCT/JP2024/044839
(87) International publication number: WO 2025/146775

(57) **Abstract**

Provided are a method for producing glucose, including contacting a liquid containing cellulose and water with a solid acid catalyst, in which the solid acid catalyst contains a carbon element, a titanium element, and silica, and has a sulfo group as a surface functional group, and a solid acid catalyst for glucose production, the solid acid catalyst containing a carbon element, a titanium element, and silica and having a sulfo group as a surface functional group.

## Description

### FIELD OF THE INVENTION

The present invention relates to a method for producing glucose and a solid acid catalyst for glucose production.

### BACKGROUND OF THE INVENTION

Bioethanol has been widely studied as a raw material for materials having a low carbon footprint in order to achieve carbon neutrality. In particular, a process including the production of bioethanol from edible biomass, the olefination of the bioethanol by a dehydration reaction, and the polymerization thereof has been studied. The technique of conversion from edible biomass to bioethanol has been established and is regarded as a promising method for producing a raw material for plastic products. However, edible biomass presents the problem of competing with food applications, and in recent years, a shift in raw material from edible biomass to non-edible biomass has been progressing as a raw material for the production of bioethanol.

Cellulose, which is non-edible biomass, can be decomposed into glucose by a hydrolysis reaction using an enzymatic method or a sulfuric acid method, and the resulting glucose can be further converted into ethanol by a fermentation method using a microorganism or the like. However, the decomposition reaction into glucose is generally accompanied by difficulties because cellulose has a chemically stable structure, and the conversion rate of cellulose and the yield of glucose are low.

In recent years, in a method for producing glucose by subjecting cellulose to a hydrolysis reaction, a solid acid catalyst has attracted attention as a hydrolysis catalyst, and various studies have been conducted. For example, as a method for producing glucose from cellulose using a solid acid catalyst, Patent Literature 1 describes "a method for producing a sugar-containing liquid containing glucose as a main component, the method including hydrolyzing cellulose in the presence of at least one type of 'catalyst for cellulose or hemicellulose hydrolysis formed of a porous carbon material having a specific surface area of 800 m²/g or more and 2500 m²/g or less and having a phenolic hydroxyl group content of 100 mmol/kg or more and 700 mmol/kg or less' and water to produce at least an oligosaccharide and glucose". In addition, Non-Patent Literature 1 describes a method for producing a saccharide such as glucose by subjecting cellulose to a hydrolysis reaction using a sulfonated silica/carbon nanocomposite. Non-Patent Literature 1 describes that this sulfonated silica/carbon nanocomposite has high conversion efficiency to glucose in the hydrolysis reaction of cellulose to glucose.

### CITATION LIST

### PATENT LITERATURE

Patent Literature 1: WO 2011/036955

### NON-PATENT LITERATURE

Non-Patent Literature 1: Green Chem., 2010, 12, 1560-1563

### SUMMARY OF THE INVENTION

### TECHNICAL PROBLEM

The yield of glucose is not sufficient even in the method described in Patent Literature 1 or in the method described in Non-Patent Literature 1, which is reported to achieve a high conversion efficiency to glucose. Furthermore, it is important to conduct research and development on methods for producing glucose from cellulose toward achieving carbon neutrality as described above, with a view toward industrialization, and in view of the feasibility of industrialization, further improvement in the yield of glucose is required.

An object of the present invention is to provide a method capable of producing glucose from cellulose in a high yield by utilizing a hydrolysis reaction of cellulose using a solid acid catalyst, and to provide a solid acid catalyst capable of producing glucose from cellulose in a high yield.

### SOLUTION TO PROBLEM

That is, the object of the present invention was achieved by the following means.
<1> A method for producing glucose, including contacting a liquid containing cellulose and water with a solid acid catalyst, in which the solid acid catalyst contains a carbon element, a titanium element, and silica, and has a sulfo group as a surface functional group.
<2> The production method according to <1>, in which the silica has mesopores.
<3> The production method according to <1> or <2>, in which the carbon element is carried on a surface of the silica.
<4> The production method according to any one of <1> to <3>, in which the titanium element is contained in the silica.
<5> A solid acid catalyst for glucose production, the catalyst containing a carbon element, a titanium element, and silica, and having a sulfo group as a surface functional group.
<6> The solid acid catalyst for glucose production according to <5>, in which the silica has mesopores.
<7>The production method according to <5> or <6>, in which the carbon element is carried on a surface of the silica.
<8> The production method according to any one of <5> to <7>, in which the titanium element is contained in the silica.

### ADVANTAGEOUS EFFECTS OF INVENTION

The present invention can provide a method and a solid acid catalyst each capable of producing glucose from cellulose in a high yield.

The above-described and other features and advantages of the present invention will appear more fully from the following description.

### BRIEF DESCRIPTION OF DRAWINGS

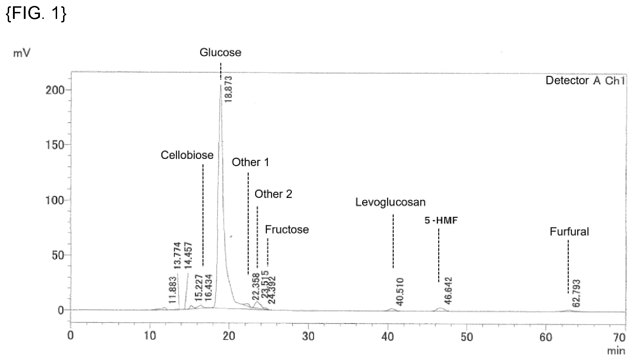

{FIG. 1}
FIG. 1 is a diagram showing an example of a chromatogram obtained by HPLC analysis of a cellulose decomposition liquid.

### DESCRIPTION OF EMBODIMENTS

A numerical value range indicated by using the term "to" in the present invention and the present specification means a range including the numerical values described before and after the term "to" as the lower limit value and the upper limit value, respectively.

### [[Method for producing glucose]]

The method for producing glucose of the present invention (hereinafter sometimes referred to as "production method of the present invention") is a method for producing glucose including contacting a liquid containing cellulose and water with a solid acid catalyst. In the production method of the present invention, as the solid acid catalyst, a solid acid catalyst containing a carbon element, a titanium element, and silica and having a sulfo group as a surface functional group (hereinafter sometimes referred to as "solid acid catalyst of the present invention") is used. Glucose can be produced in a high yield by a simple and safe step of contacting the solid acid catalyst of the present invention with cellulose. It is considered that the hydrolysis reaction is promoted by contacting cellulose with the solid acid catalyst of the present invention, resulting in an increase in the hydrolysis reaction efficiency of cellulose. Therefore, the production method of the present invention and the solid acid catalyst of the present invention are capable of producing glucose, which is a raw material for producing bioethanol important for achieving carbon neutrality, in a high yield, while effectively utilizing cellulose, which is non-edible biomass that can avoid competition with food applications, thereby also rendering industrialization feasible. As a result, the production method of the present invention and the solid acid catalyst of the present invention can be suitably applied to a method for producing bioethanol using non-edible biomass.

First, components to be used for the production method of the present invention will be explained.

### [Liquid containing cellulose and water]

In the production method of the present invention, a liquid containing cellulose and water (sometimes referred to as "reaction liquid") is used. The reaction liquid only needs to contain cellulose and water, and may contain another component. The another component may be any as long as it does not inhibit the hydrolysis reaction of cellulose, and examples thereof include hemicellulose, lignocellulose, a fatty acid, a polymeric surfactant, and aluminum sulfate.

The content of cellulose in the reaction liquid is not particularly limited, and is preferably 0.01 to 1 kg and more preferably 0.05 to 0.5 kg with respect to 1 L of water in terms of hydrolysis reaction efficiency of cellulose (hereinafter sometimes simply referred to as "hydrolysis reaction efficiency") and yield of glucose (hereinafter sometimes referred to as "glucose yield"). In the present invention, the content of cellulose in the reaction liquid refers to the substantial content of cellulose (amount in terms of cellulose) when a mixture such as waste pulp is used as a cellulose source.

The reaction liquid is usually obtained as a suspension or a dispersion by mixing cellulose and water.

### <Cellulose>

The cellulose used in the production method of the present invention may be a carbohydrate (polysaccharide) represented by the molecular formula (C₆H₁₀O₅)ₙ, and includes hemicellulose and lignocellulose in addition to cellulose. The cellulose used in the production method of the present invention may be a mixture of cellulose and hemicellulose and/or lignocellulose. However, when lignocellulose is used and when lignocellulose is contained, it is preferable to perform a lignin removal step described later.

Cellulose may be a synthetic product or a commercially available product, may be derived from non-edible biomass, or may be a waste or recovered material thereof. Examples of cellulose derived from non-edible biomass include cellulose (lignocellulose) derived from a plant such as a tree, thinned wood, or a timber, and cellulose obtained by appropriately subjecting chemical pulp, which is obtained by bleaching defatted powder or the like of a plant, to an alkali treatment. In view of the substitutability for bioethanol derived from edible biomass or the like, it is preferable to use cellulose derived from non-edible biomass as the cellulose used in the production method of the present invention. Plants are representative examples of non-edible biomass; however, it is preferable to use a waste or recovered material thereof in terms of effective utilization of resources, cost reduction, and the like. Examples of the non-edible biomass include wood powder and wood chips of a tree, thinned wood, a timber, or the like, various types of pulp (unused), and waste pulp such as board pulp and pulp sludge (paper sludge) discharged in a step of papermaking or pulp production, and pulp recovered from waste diapers. Among these, waste pulp such as pulp sludge or pulp recovered from waste diapers is particularly preferable in that the recovery amount is abundant and resources can be effectively utilized (as well as cost reduction is possible), and furthermore, poisoning of the solid acid catalyst by lignin can be avoided without performing the lignin removal step described later.

Cellulose usually exhibits crystallinity due to bonding between two or more cellulose molecules through a hydrogen bond. In the production method of the present invention, it is also possible to use cellulose exhibiting crystallinity (sometimes referred to as "crystalline cellulose"), and it is also possible to use cellulose in which the crystallinity of crystalline cellulose has been reduced by a conventional method (sometimes referred to as "low crystalline cellulose" or "microcrystalline cellulose"). The low crystalline cellulose may be one in which the crystallinity of crystalline cellulose has been partially reduced, or one in which the crystallinity has been (substantially) completely eliminated. The treatment method of reducing crystallinity is not particularly limited, and is preferably a treatment capable of cleaving the above-described hydrogen bond to at least partially generate a single-chain cellulose molecule. Cellulose at least partially containing a single-chain cellulose molecule exhibits a significantly enhanced hydrolysis reaction efficiency. Specific examples of the treatment method of reducing crystallinity include various methods described in Patent Literature 1, and among them, physical methods such as jet mill, hammer mill, ball mill, and bead mill methods are preferable.

The shape of the cellulose used in the production method of the present invention is not particularly limited, and is preferably a particle, a powder, a small piece, or the like in terms of hydrolysis reaction efficiency and glucose yield. The size of cellulose is not particularly limited, and can be appropriately determined in consideration of hydrolysis reaction efficiency and glucose yield.

### <Water>

The water used in the production method of the present invention is not particularly limited, and industrial water, well water, city water, ion-exchanged water, purified water, (ultra)pure water, and the like can be used, and ion-exchanged water, purified water, (ultra)pure water are preferable.

### [Solid acid catalyst]

The solid acid catalyst used in the production method of the present invention is a solid acid catalyst containing a carbon element, a titanium element, and silica, and having a sulfo group as a surface functional group. Glucose can be produced in a high yield by contacting the solid acid catalyst with cellulose in water. This solid acid catalyst is particularly referred to as a solid acid catalyst for glucose production when used for producing glucose from the cellulose. This solid acid catalyst has a carbon element, a titanium element, and a sulfo group (-SO₃H) on a substrate containing silica (silicon dioxide) as a main component (also referred to as "silica substrate").

The shape of the solid acid catalyst is not particularly limited, and is preferably a particle, a powder, a small piece, or the like in terms of hydrolysis reaction efficiency and glucose yield. The size (average particle diameter) of the solid acid catalyst is not particularly limited, and is preferably, for example, 0.1 to 10000 µm. The average particle diameter is a value measured by a method described in examples below.

The substrate is preferably a porous substrate (porous body), more preferably a porous body having mesopores (mesoporous body), and still more preferably mesoporous silica in that the hydrolysis reaction efficiency and glucose yield can be increased.

In the present invention, the mesopore refers to a pore having an average pore diameter of 2 to 50 nm.

When the substrate is a porous substrate, the characteristics or physical properties thereof, for example, an average pore diameter, a specific surface area, a total pore volume, and the like are not particularly limited, and can be appropriately determined. For example, the average pore diameter is preferably 2 to 10 nm and more preferably 2 to 5 nm in terms of hydrolysis reaction efficiency and glucose yield. The specific surface area is preferably 10 to 3000 m²/g and more preferably 100 to 2000 m²/g in terms of hydrolysis reaction efficiency and glucose yield. The total pore volume is preferably 0.1 to 2 mL/g and preferably 0.2 to 1 mL/g in terms of hydrolysis reaction efficiency and glucose yield. The average pore diameter, specific surface area, and total pore volume of the porous substrate are values measured by methods described in examples below.

The solid acid catalyst, usually the substrate, contains a carbon element. In the present invention, the expression that the substrate contains a carbon element means that a carbon element is present (exists) on the surface and/or inside of the substrate, and examples thereof include an aspect in which a carbon element is carried or adsorbed on the surface of the substrate (carbon element-carrying substrate), an aspect in which a carbon element is contained inside the substrate (an aspect in which the substrate is formed of a composite of silica and a carbon element), and an aspect in which a carbon element is present on both the surface and inside of the substrate. In the solid acid catalyst, the form (structure) containing a carbon element is preferably an aspect in which a carbon element is carried or adsorbed on the surface of the substrate (carbon element-carrying substrate) in that the hydrolysis reaction efficiency and glucose yield can be increased by bonding or fixing a surface functional group described later to the surface.

In the present invention, the surface of the substrate usually refers to the outer surface, but when the substrate is a porous body, the surface includes the inner surface of a pore in addition to the outer surface. On the other hand, the inside of the substrate refers to a portion not exposed on the surface.

The content of carbon element contained in the solid acid catalyst is not particularly limited, and is preferably 1 to 70 mass% and more preferably 5 to 50 mass% in the mass of the solid acid catalyst (excluding the mass of sulfo group), in that the hydrolysis reaction efficiency and glucose yield can be increased. The content of carbon element in the solid acid catalyst is a value measured by a measurement method in examples described later.

The solid acid catalyst used in the production method of the present invention, and usually the substrate, contains a titanium element. In the present invention, the expression that the substrate contains a titanium element means that a titanium element is present (exists) on the surface and/or inside of the substrate, and examples thereof include an aspect in which a titanium element is carried or adsorbed on the surface of the substrate (titanium element-carrying substrate), an aspect in which a titanium element is contained inside the substrate (an aspect in which the substrate is formed of a composite of silica and a titanium element), and an aspect in which a titanium element is present on both the surface and inside of the substrate. In the solid acid catalyst, when the substrate has a titanium element on the surface and/or inside thereof, the hydrolysis reaction efficiency and glucose yield can be increased.

The content of titanium element contained in the solid acid catalyst is not particularly limited, and is preferably 0.1 to 10 mass% and more preferably 0.5 to 5 mass% in the mass of the solid acid catalyst (excluding the mass of carbon atom and sulfo group), in that the hydrolysis reaction efficiency and glucose yield can be increased. The content of carbon element in the solid acid catalyst is a value measured by a measurement method in examples described later.

In an aspect in which the solid acid catalyst contains a carbon element and a titanium element, the aspect in which a carbon element is contained and the aspect in which a titanium element is contained can be appropriately combined; however, it is preferable that the solid acid catalyst is in a form combining an aspect in which a titanium element is contained on the surface and/or inside of the substrate and an aspect in which a carbon element is carried or adsorbed on the surface of the substrate (the solid acid catalyst in this form is also referred to as a carbon element-carrying silica-titanium composite mesoporous body), in that the hydrolysis reaction efficiency and glucose yield can be increased.

The solid acid catalyst used in the production method of the present invention has a sulfo group as a surface functional group. In the present invention, the expression that the solid acid catalyst has a sulfo group as a surface functional group means that a sulfo group is chemically bonded to the surface of the substrate and/or the carbon element. The sulfo group may be partially or entirely in the form of a salt as long as the hydrolysis reaction of cellulose can be promoted.

The content of sulfo group contained in the solid acid catalyst is not particularly limited, and is preferably 0.01 to 2 mmol/g and more preferably 0.05 to 1 mmol/g as the amount present per 1 g of the solid acid catalyst in that the hydrolysis reaction efficiency and glucose yield can be increased. The content of sulfo group in the solid acid catalyst is a value measured by a measurement method in examples described later.

As the solid acid catalyst, a commercially available product may be used, but since the solid acid catalyst has both elements of carbon and titanium and a sulfo group, it is preferable to use an appropriately synthesized one.

The method of introducing a carbon element into the substrate is not particularly limited, and various known methods can be applied. Examples of a method of carrying or adsorbing a carbon element on the surface of the substrate include a method of mixing the substrate and a carbon source and then carbonizing the carbon source, specifically, a method described in Non-Patent Literature 1. The carbon source is not particularly limited as long as it is a compound containing a carbon atom, and usually, an organic compound is used, and for example, a saccharide such as sucrose, an alcohol such as furfuryl alcohol, a hydrocarbon compound, or an alkylene oxide is preferably used.

The method of introducing a titanium element into the substrate is not particularly limited, and various known methods can be applied. Examples of a method of carrying or adsorbing a titanium element on the surface of the substrate include a method similar to the method of carrying or adsorbing a carbon element except that a titanium-containing compound is used. On the other hand, examples of a method of incorporating a titanium element on the inside or on the inside and surface of the substrate include a method of forming a substrate using a mixture of a silica precursor compound described later and a titanium-containing compound described later, for example, a sol-gel method described later.

The method of introducing a sulfo group into the substrate is not particularly limited, and various known methods can be applied. Examples thereof include a method of treating a substrate, preferably a substrate having a carbon element carried or adsorbed on the surface thereof, with sulfuric acid, specifically, a method described in Non-Patent Literature 1.

Examples of the method for producing a silica porous body as a substrate of a solid acid catalyst include a sol-gel method using a silica source (silica precursor compound) and an amphiphilic compound as a template (also referred to as "molecular template method" or "template method").

Hereinafter, a method for producing a carbon element-carrying silica-titanium composite mesoporous body having a sulfo group as a surface functional group, which is suitable as a solid acid catalyst, by a sol-gel method will be described as an example.

A general sol-gel method is, for example, a method in which a silica precursor compound is subjected to a sol-gel reaction (hydrolysis reaction and condensation reaction) around self-assembled micellar particles formed of a cationic surfactant to form an inorganic-organic nanocomposite, which is then fired or subjected to an acid treatment (removal of cationic surfactant).

In the preferable solid acid catalyst, first, a silica-titanium composite mesoporous body in which a titanium element is contained on the surface and/or inside of the substrate is produced using the sol-gel method. At this time, the production can be performed in the same manner as in a general sol-gel method except that a titanium precursor compound described later is used together with the silica precursor compound (the silica precursor compound and the titanium precursor compound are allowed to coexist). Therefore, the type of each step, the operation and reaction conditions in each step, and the like in the sol-gel method to be applied when the silica-titanium composite mesoporous body is produced, can be appropriately determined with reference to a general sol-gel method. Reference may be made to, for example, the method described in Non-Patent Literature 1, a series of steps in a synthesis method in examples described later, the operation and reaction conditions in each step, and the like.

As is the case with a general sol-gel method, in the above-described sol-gel method, the characteristics or physical properties of the porous body, such as the size (average pore diameter) of mesopores and the shape of mesopores, as well as the skeletal shape of the porous body, can be adjusted or changed by changing the type of the silica precursor compound, in particular the type of the amphiphilic compound, or the preparation conditions such as the solvent composition, temperature, and time.

As the amphiphilic compound used as a template, a compound usually used in a general sol-gel method can be used without particular limitation, and usually, an amphiphilic surfactant is used, and a cationic surfactant, a block copolymer, or the like is suitably used. In the present invention, it is preferable to use a cationic surfactant as a template in that the average pore diameter of mesopores can be set to be relatively small, and the hydrolysis reaction efficiency and glucose yield can be further increased. As the cationic surfactant, a surfactant usually used as a template in a sol-gel method can be used without particular limitation, and examples thereof include an ammonium-based cationic surfactant. As the ammonium-based cationic surfactant, an alkylammonium salt is preferable, a tetraalkylammonium salt is preferable, an ammonium salt containing a long chain (having 6 or more carbon atoms) linear alkyl group is more preferable, and a mono-long chain linear alkyl tri-short chain (having 4 or less carbon atoms) alkylammonium salt is particularly preferable. An anion forming a quaternary ammonium salt is not particularly limited, and examples thereof include inorganic anions such as a hydroxide ion, a halide ion, and a perhalogenate ion.

In addition, MCM-41 and the like are representative of a silica mesoporous body prepared by a sol-gel method using a cationic surfactant, and SBA-15 and the like are representative of a silica porous body prepared by a sol-gel method using a block copolymer.

As the silica precursor compound, a compound usually used in a general sol-gel method can be used without particular limitation, and examples thereof include halogen compounds, hydroxides, alkoxides, and the like of silicon, and alkoxides and/or alkyl alkoxides of silicon are preferable. The number of carbon atoms in the alkyl group forming the alkyl alkoxide is not particularly limited, and is preferably 1 to 6, and more preferably 1 to 3.

In the sol-gel method, a titanium-containing compound is used together with the silica precursor compound. Examples of the titanium-containing compound include a titanium precursor compound which is converted into a titanium element together with the silica precursor compound by a sol-gel method. As the titanium precursor compound, a halogen compound, a hydroxide, an alkoxide, and the like of titanium are preferable, and an alkoxide of titanium and/or an alkyl alkoxide of titanium (alkoxytitanium) are more preferable. The number of carbon atoms in the alkyl group forming the alkyl alkoxide is not particularly limited, and is preferably 1 to 6, and more preferably 1 to 3.

Next, a carbon element is carried or adsorbed on the surface of the obtained silica-titanium composite mesoporous body. The method of carrying or adsorbing a carbon element is not particularly limited, and is as described above.

A sulfo group is introduced as a surface functional group into the carbon element-carrying silica-titanium composite mesoporous body thus obtained. The method of introducing a sulfo group is not particularly limited, and is as described above.

The silica-titanium composite mesoporous body, the carbon element-carrying silica-titanium composite mesoporous body, and/or the element-carrying silica-titanium composite mesoporous body into which a sulfo group is introduced can also be appropriately pulverized and crushed.

### [Step of contacting liquid containing cellulose and water with solid acid catalyst]

The production method of the present invention includes contacting a liquid containing cellulose and water (reaction liquid) with the solid acid catalyst of the present invention, and by this contacting, cellulose can be subjected to a hydrolysis reaction in the presence of the solid acid catalyst and converted into glucose.

Each component used in this contacting step may be of one type or of two or more types.

The hydrolysis reaction of cellulose that occurs in the contacting step is generally considered to involve a hydrolysis reaction from cellulose to an oligosaccharide and a hydrolysis reaction from an oligosaccharide to glucose. Therefore, in the contacting step, a sugar-containing liquid (also referred to as "saccharified liquid" or "cellulose decomposition product") containing glucose is obtained as a hydrolysis treatment (saccharification treatment) of cellulose. This sugar-containing liquid usually contains, in addition to water and the solid acid catalyst, glucose as a main component and contains other components. Examples of other components which may be contained in the sugar-containing liquid include a partial hydrolysis product of cellulose and a reaction product of glucose, and examples thereof include saccharides such as tetramer or higher oligosaccharides, cellotriose, cellobiose, mannose, fructose, and levoglucosan, and 5-hydroxymethylfurfural and furfural. In the present invention, the main component refers to a component having the largest content by mass among the components contained.

In the contacting step using the solid acid catalyst of the present invention, the hydrolysis reaction efficiency of cellulose is high, and the occurrence and promotion of each side reaction with respect to the hydrolysis reaction to glucose can be suppressed, so that glucose can be obtained at a high selectivity in a high yield. Therefore, the sugar-containing liquid obtained in the contacting step contains glucose as a main component and contains other components at low contents. For example, as an example of the sugar-containing liquid, a sugar-containing liquid containing glucose as a main component and containing cellobiose, fructose, levoglucosan, 5-hydroxymethylfurfural (HMF), and furfural at a low total content can be mentioned.

A suitable sugar-containing liquid obtained in the contacting step is a sugar-containing liquid having a composition in which the total area value of peaks derived from components having a molecular weight lower than that of glucose is 0.01 to 0.2 times the area value of a peak derived from glucose when subjected to LC analysis with a suggested refractive index detector. When the area ratio of the total area value to the area value of the peak derived from glucose (hereinafter sometimes referred to as "component area ratio") is 0.01 to 0.2 times, it can be seen that cellulose can be converted into glucose at a high selectivity in the contacting step. In such a suitable sugar-containing liquid, its composition, characteristics, and the like are improved, and use thereof as a raw material liquid for ethanol fermentation of glucose can suppress the occurrence or promotion of side reactions to allow ethanol fermentation of glucose to preferentially proceed as a main reaction. In addition, even if acetic acid bacteria are present, the growth thereof and acetic acid fermentation can be highly suppressed, and the amount and selectivity (content) of ethanol obtained by ethanol fermentation can be further enhanced. The component area ratio is preferably 0.05 to 0.2 times and more preferably 0.1 to 0.2 times in that ethanol can be produced in a high yield at a high selectivity by further increasing the efficiency of ethanol fermentation and further suppressing the growth of acetic acid bacteria and acetic acid fermentation. Here, the component area ratio is a value measured by a method described in examples below.

A suitable sugar-containing liquid may contain the above-described partial hydrolysis product of cellulose, a reaction product such as glucose, and the like, but satisfies the above-described component area ratio for a component having a lower molecular weight than glucose (sometimes referred to as a "low molecular weight component") identified by LC analysis. The low molecular weight component cannot be unequivocally determined due to variations in the origin and composition of cellulose used in the hydrolysis reaction, the contact conditions between glucose and the solid acid catalyst, and the like, but generally includes components having a lower molecular weight than glucose among the partial hydrolysis products of cellulose, reaction products such as glucose, and the like. Examples thereof include levoglucosan, HMF, and furfural, and further include unidentifiable components, and levoglucosan, HMF, and furfural are preferable.

The content of glucose in a suitable sugar-containing liquid cannot be unequivocally determined as described above, but is a content at which at least the component area ratio is 0.01 to 0.2 times, and is a content falling within the above preferable range or the above more preferable range. The content of glucose in a suitable sugar-containing liquid, expressed as a mass ratio in all components contained in the cellulose decomposition composition, is, for example, preferably 50 to 99 mass%, more preferably 83 to 99 mass% in that ethanol can be efficiently produced.

In the production method of the present invention, glucose can be obtained as a main component by efficiently hydrolyzing cellulose. At this time, the conversion rate of cellulose, the selectivity of glucose (the mass ratio of the content of glucose to the content of other components), and the like vary depending on the type of cellulose, the type and use amount of the solid acid catalyst, the hydrolysis reaction conditions, and the like, and thus cannot be unequivocally determined, but at least a yield of glucose of 45% or more can be achieved.

In the production method of the present invention, a liquid containing cellulose and water (reaction liquid) is contacted with a solid acid catalyst under appropriate reaction conditions usually under heating.

The contacting method may be a method capable of contacting three components of cellulose, water, and a solid acid catalyst, and examples thereof include a method of contacting a reaction liquid prepared in advance with a solid acid catalyst, and a method of contacting cellulose, water, and a solid acid catalyst without preparing a reaction liquid in advance (a method of contacting cellulose and a solid acid catalyst in the presence of water). In the contacting step in the present invention, references to, for example, contacting the reaction liquid with, or adding it to, the solid acid catalyst encompass an aspect in which, in place of the reaction liquid, cellulose and water are, for example, separately contacted with or added to the solid acid catalyst.

In the contacting step, for example, the reaction liquid and the solid acid catalyst are added to a sealed vessel, followed by heating. The heating temperature (reaction temperature) of the hydrolysis reaction is not particularly limited, and can be set to, for example, 110 to 200°C. The heating temperature is preferably 120 to 180°C, and more preferably 120 to 150°C in that the yield of glucose can be increased by increasing the hydrolysis reaction efficiency of cellulose and suppressing by-production of other components. The heating time (reaction time) is appropriately determined according to the heating temperature, the hydrolysis reaction efficiency (conversion rate) of cellulose, the glucose yield, and the like, and can be set to, for example, 1 to 48 hours. The inside of the sealed vessel under heating may be at normal pressure, but is usually in a pressurized state where the partial pressure of water vapor exceeds, for example, 0.1 MPa. In the present invention, the hydrolysis reaction can also be performed under a positively pressurized environment. The pressure at this time is not particularly limited, and can be, for example, more than 0.1 MPa and 20 MPa or less, and is preferably 0.1 to 10 MPa. The reaction environment (atmosphere) is not particularly limited, and may be an inert gas atmosphere or the like, but may be an air atmosphere or a water vapor atmosphere. The reaction environment is preferably an air atmosphere and/or a water vapor atmosphere in view of industrial production.

The amounts of the reaction liquid and the solid acid catalyst used are not particularly limited, and at least the amount of water in the reaction system (the amount of water present) is equal to or more than the amount required for the hydrolysis reaction of cellulose. The amount of water in the reaction system is, for example, preferably 0.1 to 1000 mass times, more preferably 1 to 100 mass times the amount of cellulose present in terms of mixing property (stirring property) and handleability of the reaction system, and further hydrolysis reaction efficiency and glucose yield. In the contacting step, water can also be added separately from the reaction liquid, and the amount of water in the reaction system refers to the amount of water derived from the reaction liquid. When water is added, the amount of water is the total amount of water derived from the reaction liquid and the added water.

The amount of the solid acid catalyst used can be appropriately determined in consideration of reaction conditions, hydrolysis reaction efficiency, glucose yield, and the like, and is, for example, preferably 0.01 to 10 mass times, more preferably 0.1 to 5 mass times the amount of cellulose present.

The contacting step can be performed by a batch method using a sealed vessel such as an autoclave, or can be performed by a continuous method using a reaction tube filled with the solid acid catalyst. In the batch method, it is preferable to mix the reaction liquid and the solid acid catalyst by an appropriate means such as stirring or shaking in terms of hydrolysis reaction efficiency and glucose yield. Examples of the continuous method include a method in which the reaction liquid is continuously or intermittently allowed to flow in a heated reaction tube.

As described above, a mixture of a sugar-containing liquid containing glucose as a main component and a solid acid catalyst (also referred to as reaction mixture) can be obtained by subjecting cellulose to a hydrolysis reaction in the presence of the solid acid catalyst.

### [Post-treatment step]

In the production method of the present invention, glucose can be obtained by subjecting the reaction mixture to a post-treatment after the above-described contacting step. Examples of the post-treatment of the reaction mixture include a step of cooling the reaction mixture and a step of subjecting the reaction mixture to solid-liquid separation.

The reaction mixture can also be subjected to solid-liquid separation without cooling, but it is preferable to cool the reaction mixture in order to suppress further reaction of glucose or in consideration of workability, safety, and the like. The cooling of the reaction mixture may be performed by natural cooling, or by using various refrigerants. The cooling temperature for the reaction mixture is not particularly limited, and can be usually 100°C or lower, and is preferably 80°C or lower in that a high yield of glucose can be maintained by suppressing a further reaction of glucose, and is preferably near room temperature (for example, 15 to 40°C) in consideration of workability, safety, and the like. The lower limit temperature of the cooling temperature can be, for example, 0°C or higher, and is preferably 15°C or higher in terms of workability. The cooling rate at this time can be appropriately determined, and it is preferable to lower the temperature by 0.1 to 10°C per minute. The cooling time can be appropriately determined according to the heating temperature, the cooling rate, and the like.

The appropriately cooled reaction mixture is subjected to solid-liquid separation to separate the sugar-containing liquid containing glucose as a main component (cellulose decomposition liquid) as a liquid phase and at least the solid acid catalyst and unreacted cellulose as a solid phase. The solid-liquid separation method is not particularly limited, and examples thereof include a filtration method, a centrifugal separation method, and a precipitation method.

### [Other steps]

In the production method of the present invention, steps other than the contacting step and the post-treatment step can also be performed. Examples thereof include a step of isolating and purifying glucose from the separated and recovered sugar-containing liquid, a step of isolating the solid acid catalyst and unreacted cellulose from the separated and recovered solid phase, a step of washing and regenerating the isolated solid acid catalyst, a step of removing impurities, contaminants, and the like in a waste when the above-described waste is used as the cellulose source, and a step of removing lignin in cellulose.

As a method of isolating and purifying glucose, various known isolation methods and various known purification methods can be applied without particular limitation. In addition, the solid acid catalyst used in the present invention can be reused in a separated and recovered state (for example, as a mixture with unreacted cellulose), and it is not necessary to perform an isolation step, a washing step, and a regeneration step of the solid acid catalyst. However, since the catalytic activity of the solid acid catalyst usually gradually decreases as the number of uses increases, the solid acid catalyst can also be washed and/or regenerated in consideration of, for example, a decrease in catalytic activity, hydrolysis reaction efficiency or glucose yield. The washing and regenerating methods are not particularly limited.

In the production method of the present invention, glucose can be obtained from cellulose in a high yield in a relatively short time by a simple and safe step of contacting the reaction liquid with the solid acid catalyst of the present invention. In addition, the post-treatment step of the contacting step is also simple and safe, and the solid acid catalyst separated and recovered can be reused. Furthermore, since the occurrence and promotion of each side reaction can be suppressed in the hydrolysis reaction of cellulose, glucose can also be produced at high selectivity (high purity). As a result, the production method of the present invention and the solid acid catalyst of the present invention are suitable as a method for obtaining a raw material for producing bioethanol using non-edible biomass as a raw material. In addition, when waste pulp is used as the cellulose source, the hydrolysis reaction efficiency of cellulose and the yield of glucose can be further increased, and the non-edible biomass resource can also be effectively utilized. Therefore, the production method of the present invention and the solid acid catalyst of the present invention are capable of efficiently producing glucose from cellulose at low cost, and are also suitable for industrialization of a hydrolysis reaction of cellulose (glucose production method) and a method for producing bioethanol.

### EXAMPLES

Hereinafter, the present invention will be described in more detail with reference to examples, but the present invention is not limited to these examples.

### [Preparation of solid acid catalyst]

### <Experimental Example 1-1. Preparation of Ti element-containing solid acid catalyst (i) for glucose production>

### (1) Preparation of silica porous body (i)

### (First step)

A 16 mass% hexadecyltrimethylammonium hydroxide aqueous solution (625.5 g) was stirred, and a mixed solution of 9.25 g of tetraisopropyl titanate and 50.0 g of 2-propanol was added dropwise thereto at room temperature (25°C). After stirring for 30 minutes, 190.5 g of tetramethyl orthosilicate was added dropwise thereto. After 5.0 g of 2-propanol was added thereto, stirring was continued for 3 hours, and as a result, a precipitate was generated.

### (Second step)

The resulting precipitate was separated by filtration and washed with 5 L of ion-exchanged water. The obtained washed solid was dried at 100°C for 5 hours under reduced pressure.

### (Third step)

In a flask, 20 g of the dried solid obtained in the second step was placed, and a mixed solution of 200 mL of methanol and 10 g of concentrated hydrochloric acid (content: 36 mass%) was added thereto (acid treatment). Heating was continued at a reflux temperature for 1 hour while stirring, the mixture was allowed to cool, and then the liquid phase was removed by filtration. The same operation was repeated again using a mixed liquid of 200 mL of methanol and 5 g of concentrated hydrochloric acid. Finally, the resultant was refluxed with 200 mL of methanol for 1 hour, and finally, the solid separated by filtration was dried under reduced pressure at 120°C and 10 mmHg for 1.5 hours.

### (Fourth step)

The solid obtained in the third step was subjected to a heat treatment (fired) at 600°C for 3 hours under air flow.

### (Fifth step)

The fired solid obtained in the fourth step was pulverized 8 times with a hammer mill to obtain a powdery silica porous body (i) having an average particle diameter of 10 µm.

The average particle diameter was measured using ion-exchanged water as a dispersion medium with a laser diffraction/scattering particle size distribution analyzer (LA-950 manufactured by HORIBA, Ltd.), and calculated on a volume basis.

The powdery silica porous body (i) prepared as described above was a silica-titanium composite mesoporous body, and had a specific surface area of 1160 m²/g, a total pore volume of 0.60 mL/g, and an average pore diameter of 2.1 nm.

The specific surface area and total pore volume were determined by measuring the nitrogen adsorption isotherm at liquid nitrogen temperature using BELSORP MINI X (manufactured by MicrotracBEL Corp.) after degassing the silica porous body (i) under vacuum at 120°C for 2 hours, and calculating the values by the BET method and from the value of the nitrogen adsorption amount in the vicinity of the relative pressure (P/P0 = 0.96), respectively. In addition, the average pore diameter was calculated from the value of the total pore volume and the value of the specific surface area by the following (Formula 1). (Formula 1): 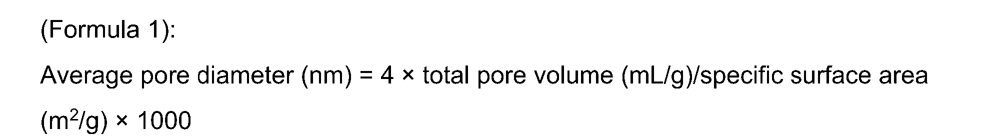 Average pore diameter (nm) = 4 × total pore volume (mL/g)/specific surface area (m2/g) × 1000

The Ti concentration in the powdery silica porous body (i) prepared as described above was analyzed by XRF and found to be 1.5 mass%.

### (2) Preparation of silica porous body-carbon composite (i)

In 2.5 g of the silica porous body (i), furfuryl alcohol (FA) as a carbon source was impregnated under vacuum into the pores of the silica porous body at room temperature. After the impregnation, FA on the outer surface of the silica porous body was washed with mesitylene, followed by heating at 150°C for 24 hours to polymerize FA in the pores. The solid separated by filtration after polymerization was dried under reduced pressure at 120°C for 12 hours. The obtained solid was subjected to a heat treatment at 550°C for 3 hours in a nitrogen atmosphere to carbonize the polymerized FA in the pores to prepare a silica porous body-carbon composite (i) as a carbon element-carrying silica-titanium composite mesoporous body.

### (3) Preparation of solid acid catalyst (i)

To an autoclave, 1 g of the silica porous body-carbon composite (i) and 20 mL of 95 mass% sulfuric acid were added and treated at 150°C for 15 hours. After the treatment, washing with water was performed at 50°C until the pH of the washing liquid exhibited the same value as that of purified water. After washing with water, the solid separated by filtration was dried under reduced pressure at 120°C for 12 hours to obtain 1 g of a solid acid catalyst (i) as a carbon element-carrying silica-titanium composite mesoporous body having a sulfo group as a surface functional group.

### <Experimental Example 1-2. Preparation of Ti element-free solid acid catalyst (ii)>

### (1) Preparation of silica porous body-carbon composite (ii)

A solid acid catalyst (ii) was prepared using the same method as described in Non-Patent Literature 1 (average pore diameter: 6 to 8 nm (literature value)).

The preparation method is shown below.

To a solution obtained by mixing 32 g of ethanol and 0.3 g of concentrated hydrochloric acid, 6.4 g of Pluronic F127 (reagent manufactured by FUJIFILM Wako Pure Chemical Corporation) as a surfactant serving as a template was added, and the mixture was subjected to an ultrasonic treatment for 1 hour. Next, 8.32 g of tetraethoxysilane (TEOS) as a silica source and 10 g of a sucrose aqueous solution as a carbon source were added thereto, and the resulting mixture was treated again with ultrasonic waves for 1 hour. The mass ratio between the mass of Si element contained in TEOS and C element contained in sucrose was as follows: Si:C = 33:66. Thereafter, heating was performed at 40°C for 20 hours to evaporate ethanol, and heating was performed at 160°C for 24 hours to perform thermal polymerization. The solid obtained after the thermal polymerization was carbonized by a heat treatment at 550°C for 3 hours in a nitrogen atmosphere in an electric furnace to prepare a silica porous body-carbon composite (ii).

### (2) Preparation of solid acid catalyst (ii)

To an autoclave, 1 g of the silica porous body-carbon composite (ii) and 20 mL of 95 mass% sulfuric acid were added and treated at 150°C for 24 hours. After the treatment, washing with water was performed at 50°C until the pH of the washing liquid exhibited the same value as that of purified water. After washing with water, the solid separated by filtration was dried under reduced pressure at 120°C for 12 hours to obtain a solid acid catalyst (ii).

### <Experimental Example 1-3. Preparation of Ti element-free solid acid catalyst (iii)>

### (1) Preparation of silica porous body-carbon composite (iii)

As the silica porous body, commercially available MCM-41 (reagent manufactured by Sigma-Aldrich Co. LLC, Ti element-free, average pore diameter 2.1 to 2.7 nm (catalog value)) was used. In 2.5 g of MCM-41, furfuryl alcohol (FA) as a carbon source was impregnated under vacuum into the pores of MCM-41 at room temperature. After the impregnation, FA on the outer surface of MCM-41 was washed with mesitylene, followed by heating at 150°C for 24 hours to polymerize FA in the pores. The solid separated by filtration after polymerization was dried under reduced pressure at 120°C for 12 hours. The obtained solid was subjected to a heat treatment at 550°C for 3 hours in a nitrogen atmosphere to carbonize the polymerized FA in the pores to prepare a silica porous body-carbon composite (iii).

### (2) Preparation of solid acid catalyst (iii)

To an autoclave, 1 g of the silica porous body-carbon composite and 20 mL of 95 mass% sulfuric acid were added and treated at 150°C for 24 hours. After the treatment, washing with water was performed at 50°C until the pH of the washing liquid exhibited the same value as that of purified water. After washing with water, the solid separated by filtration was dried under reduced pressure at 120°C for 12 hours to obtain a solid acid catalyst (iii).

### <Experimental Example 2. Measurement of carbon content of silica porous body-carbon composite>

The carbon content of each of the silica porous body-carbon composites (i) to (iii) was determined from the weight reduction rate by thermogravimetric analysis. The thermogravimetric analysis was performed in an air atmosphere at a furnace temperature increased to 1000°C at 5°C/min. The results are shown in Table 1.

### [Table 1]

**Table 1**

| | Carbon content (mass%) |
|---|---|
| Silica porous body-carbon composite (i) | 24 |
| Silica porous body-carbon composite (ii) | 40 |
| Silica porous body-carbon composite (iii) | 5 |

### <Experimental Example 3. Measurement of sulfo group content of solid acid catalyst>

The sulfo group contents of the solid acid catalysts (i) and (ii) were measured by a titration method using an automatic potentiometric titrator (manufactured by Kyoto Electronics Co., Ltd.). Each 50 mg portion of the solid acid catalyst was weighed into a screw tube bottle, and 30 mL of a NaCl aqueous solution prepared to 0.05 N was added using a volumetric pipette, followed by stirring with a stirrer for 15 hours. After stirring, the solution in the screw tube bottle was filtered, and 10 mL of the filtrate was collected using a volumetric pipette and transferred to a 50 mL beaker. In the 50 mL beaker, 2 drops of a phenolphthalein solution were added with a Pasteur pipette, and titration was performed by dropwise addition of a 0.005 N NaOH aqueous solution in portions of 0.1 mL. The Na⁺ exchange amount was calculated from the equivalent point of the titration result to determine the sulfo group content. The results are shown in Table 2.

### [Table 2]

**Table 2**

| | Sulfo group content (mmol/g) |
|---|---|
| Solid acid catalyst (i) | 0.09 |
| Solid acid catalyst (ii) | 0.14 |
| Solid acid catalyst (iii) | 0.37 (Literature value) |

### <Example 1>

### (1) Hydrolysis reaction of cellulose

As the cellulose as the reaction substrate, one treated as follows was used.

In a ceramic pot mill, 1 kg of zirconia balls having a diameter of 1 cm and 5 g of microcrystalline cellulose (Avicel PH-101, reagent manufactured by Sigma-Aldrich Co. LLC.) were placed. The pot mill was set on a tabletop pot mill rotary table, and a ball milling treatment was performed at 200 rpm for 120 hours.

To an autoclave equipped with a stirring device, 50 mg of the ball-milled cellulose, 50 mg of the solid acid catalyst (i), and 5 mL of purified water were added, and the temperature was raised from room temperature to 150°C in about 30 minutes while stirring at 300 rpm, and then a hydrolysis reaction of the cellulose was performed at 150°C for 24 hours (internal pressure: 0.5 MPa). After completion of the reaction, the autoclave was allowed to cool to room temperature. Thereafter, the reaction liquid was filtered and separated into a liquid and a solid to obtain a cellulose decomposition liquid (i) and a solid residue (i).

### (2) High-performance liquid chromatography (HPLC) analysis

The cellulose decomposition liquid (i) was subjected to HPLC analysis, and the glucose concentration (mass%) in the cellulose decomposition liquid (i) was quantified by an absolute calibration curve method using a glucose reagent. HPLC analysis was performed with LC-20A (manufactured by Shimadzu Corporation) using Shodex SP0810 (manufactured by Resonac Corporation, inner diameter: 8 mm, length: 300 mm) as a column, purified water (0.5 mL/min) as a mobile phase, and a differential refractive index detector (RID-20A) as a detector at a column temperature of 70°C, a sample injection amount of 20 µL, an analysis time of 70 minutes, and a sampling rate of 100 ms.

### (3) Measurement of amount of solid residue

A value obtained by subtracting the mass of the added solid acid catalyst from the mass measured after drying the solid residue (i) at 100°C for 12 hours was determined as the mass of unreacted cellulose.

### (4) Calculation of glucose yield

From the amount of glucose obtained calculated from the following (Formula 2) and the amount of cellulose charged, the glucose yield was determined by the following (Formula 3). (Formula 2): 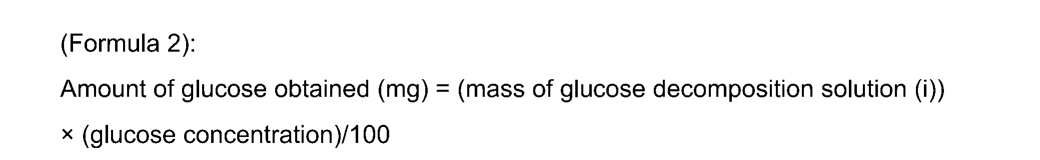 Amount of glucose obtained (mg) = (mass of glucose decomposition solution (i)) × (glucose concentration)/100

Here, the glucose concentration used in (Formula 2) is the glucose concentration (mass%) measured in the above (2). (Formula 3): 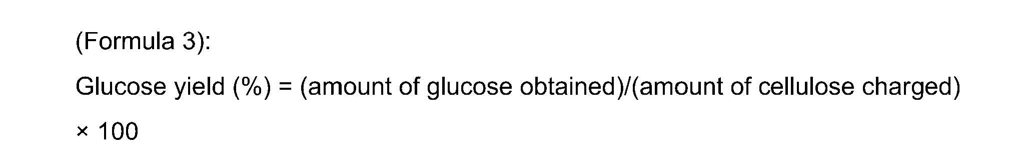 Glucose yield (%) = (amount of glucose obtained)/(amount of cellulose charged) × 100

The component area ratio of the cellulose decomposition liquid is calculated as follows using a chromatogram obtained by HPLC analysis of the cellulose decomposition liquid.

An example of the chromatogram obtained by HPLC analysis of the cellulose decomposition liquid is shown in FIG. 1. For the component area ratio of the cellulose decomposition liquid, the type of each component in the cellulose decomposition product is identified using the chromatogram. Specifically, each component detected in the chromatogram was identified by, for example, comparing with the retention time of each compound reagent in the column: Shodex SP0810.

Next, peak area values derived from glucose and components having a lower molecular weight than glucose that elute after the retention time of glucose in the obtained chromatogram are obtained by the following waveform processing parameter treatment. The waveform processing parameters used for peak detection are set to a slope of 200 µV/min, a width of 5 sec, and a minimum area of 1000 counts as the minimum area/height, under which conditions the area values are calculated.

The component area ratio is calculated using the area value of each component thus obtained by dividing the peak area value derived from glucose by the total value of the peak area values derived from low molecular weight components.

The component area ratio of the cellulose decomposition liquid (i) calculated by the above method was within a range of 0.01 to 0.2 times.

### <Comparative Example 1>

The glucose yield was determined in the same manner as in Example 1 except that the hydrolysis reaction of cellulose was performed without adding the solid acid catalyst.

### <Comparative Example 2>

The glucose yield was determined in the same manner as in Example 1 except that the solid acid catalyst (ii) was used instead of the solid acid catalyst (i) in the hydrolysis reaction of cellulose.

### <Comparative Example 3>

The glucose yield was determined in the same manner as in Example 1 except that the solid acid catalyst (iii) was used instead of the solid acid catalyst (i) in the hydrolysis reaction of cellulose.

The results of the hydrolysis reaction of cellulose in Example 1 and Comparative Examples 1 to 3 are shown in Table 3.

### [Table 3]

**Table 3**

| | Solid acid catalyst | Containing Ti element | Glucose yield (%) |
|---|---|---|---|
| Ex. 1 | Solid acid catalyst (i) | Yes | 70 |
| CEx. 1 | None | - | 5 |
| CEx. 2 | Solid acid catalyst (ii) | No | 42 |
| CEx. 3 | Solid acid catalyst (iii) | No | 13 |

| | | | |
|---|---|---|---|
| Remarks: 'Ex.' means Example according to this invention, and 'CEx.' means Comparative Example | | | |

It was found that Comparative Example 1, in which the solid acid catalyst was not used, and Comparative Examples 2 and 3, in which the solid acid catalyst (ii) or (iii) containing no Ti element was used, exhibited a low glucose yield and were not sufficient for industrial use.

On the other hand, Example 1, in which the solid acid catalyst (i) containing the Ti element was used, exhibited a high glucose yield of 70%. In Example 1, the hydrolysis reaction was performed using commercially available cellulose, and from this result, it is found that a similar result can be obtained even when cellulose derived from waste pulp is used. In the present invention, although the detailed reason why a high glucose yield can be obtained has not yet been clarified, it is presumed that, due to the catalytic action of the Ti element contained in the silica porous body (i), the carbon coating layer formed during preparation of the silica porous body-carbon composite (i) has a structure suitable for the hydrolysis reaction of cellulose.

As described above, the production method of the present invention and the solid acid catalyst of the present invention are capable of producing glucose from cellulose at low cost by a simple and safe step in a high yield sufficient for industrial production and preferably at a high selectivity (high purity). Therefore, it can be seen that the production method of the present invention and the solid acid catalyst of the present invention are suitable as a method and a solid acid catalyst for obtaining a raw material for producing bioethanol using non-edible biomass as a raw material, and are suitable for industrial production for a hydrolysis reaction of cellulose and a method for producing bioethanol.

Having described the present invention as related to the embodiment, it is our intention that the present invention not be limited by any of the details of the description, unless otherwise specified, but rather be construed broadly within its spirit and scope as set out in the accompanying claims.

The present application claims priority of Patent Application No. 2024-000266 filed in Japan on January 4, 2024, which is herein incorporated by reference as part of the present specification.

## Claims

1. A method for producing glucose, the method comprising contacting a liquid containing cellulose and water with a solid acid catalyst,
wherein the solid acid catalyst contains a carbon element, a titanium element, and silica, and has a sulfo group as a surface functional group.

2. The production method according to claim 1, wherein the silica has mesopores.

3. The production method according to claim 1 or 2, wherein the carbon element is carried on a surface of the silica.

4. The production method according to claim 1 or 2, wherein the titanium element is contained in the silica.

5. The production method according to claim 3, wherein the titanium element is contained in the silica.

6. A solid acid catalyst for glucose production, the catalyst comprising a carbon element, a titanium element, and silica, and having a sulfo group as a surface functional group.

7. The solid acid catalyst for glucose production according to claim 6, wherein the silica has mesopores.

8. The solid acid catalyst for glucose production according to claim 6 or 7, wherein the carbon element is carried on a surface of the silica.

9. The solid acid catalyst for glucose production according to claim 6 or 7, wherein the titanium element is contained in the silica.

10. The solid acid catalyst for glucose production according to claim 8, wherein the titanium element is contained in the silica.
